(19) Europäisches Patentamt
European Patent Office
Office européen des brevets

(11) Veröffentlichungsnummer: **0 118 787**
**A1**

(12) # EUROPÄISCHE PATENTANMELDUNG

(21) Anmeldenummer: **84101489.7**

(22) Anmeldetag: **14.02.84**

(51) Int. Cl.³: **C 07 D 233/64**
A 61 K 31/415

(30) Priorität: **15.02.83 GB 8304182**
**02.11.83 GB 8329238**

(43) Veröffentlichungstag der Anmeldung:
**19.09.84 Patentblatt 84/38**

(84) Benannte Vertragsstaaten:
**AT BE CH DE FR GB IT LI LU NL SE**

(71) Anmelder: **F. HOFFMANN-LA ROCHE & CO.**
**Aktiengesellschaft**

**CH-4002 Basel(CH)**

(72) Erfinder: **Cashin, Colin Henry**
**"Thurston" Church Lane**
**Preston near Hitchin Herts(GB)**

(72) Erfinder: **Roberts, Noel Allan**
**19 Elliswick Road**
**Harpenden, Herts(GB)**

(72) Erfinder: **Tong, Brian Peter**
**7 Dalkeith Road**
**Harpenden, Herts(GB)**

(74) Vertreter: **Lederer, Franz, Dr. et al,**
**Patentanwälte Dr. Franz Lederer Dipl.-Ing. Reiner F.**
**Meyer-Roxlau Lucile-Grahn-Strasse 22**
**D-8000 München 80(DE)**

(54) **Alpha-Amino-2-imidazolpropionsäurederivate.**

(57) Die vorliegende Erfindung betrifft Imidazolderivate der allgemeinen Formel

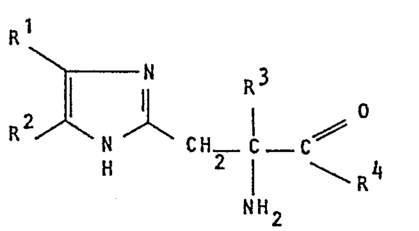

I

worin $R^1$, $R^2$ and $R^3$ je Wasserstoff oder niederes Alkyl und $R^4$ Hydroxy, niederes Alkoxy, Aryl-niederes Alkoxy, Amino, Mono(niederes Alkyl)amino oder Di(niederes Alkyl)amino bedeuten, und pharmazeutisch annehmbare Salze davon, welche zur Behandlung von degenerativen Gelenkkrankeiten und der Wilson-Krankheit verwendet werden können. Mit Ausnahme der D,L-Verbindungen der Formel I, worin $R^1$, $R^2$ und $R^3$ je Wasserstoff und $R^4$ Hydroxy oder Methoxy bedeuten, sind diese Verbindungen neu. Ein Verfahren zur Herstellung dieser neuen Verbindungen der Formel I ist ebenfalls beschrieben.

EP 0 118 787 A1

Croydon Printing Company Ltd.

F.HOFFMANN-LA ROCHE & CO. Aktiengesellschaft, Basel/Schweiz 0118787

RAN 4070/65

# α-Amino-2-imidazolpropionsäurederivate

Die vorliegende Erfindung betrifft Imidazolderivate und diese enthaltende pharmazeutische Präparate.

Die erfindungsgemäss bereitgestellten pharmazeutischen Präparate enthalten als Wirkstoff eine D,L-, D- oder L-Verbindung der allgemeinen Formel

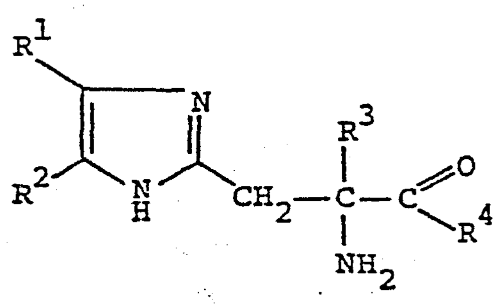

I

worin $R^1$, $R^2$ und $R^3$ je Wasserstoff oder niederes Alkyl und $R^4$ Hydroxy, niederes Alkoxy, Aryl-niederes Alkoxy, Amino, Mono(niederes Alkyl)amino oder Di(niederes Alkyl)amino bedeuten,

oder ein pharmazeutisch annehmbares Salz davon.

Die erfindungsgemässen pharmazeutischen Präparate eignen sich zur Behandlung von degenerativen Gelenkerkrankungen, wie der rheumatoiden Arthritis und der Osteoarthritis, sowie der Wilson-Krankheit.

Nt/19.12.1983

Der in der vorliegenden Beschreibung verwendete Ausdruck "niederes Alkyl" für sich allein genommen oder in Kombinationen, wie in "Mono(niederes Alkyl)amino" und "Di(niederes Alkyl)amino", bezeichnet eine geradkettige oder verzweigte Alkylgruppe mit bis zu 4 Kohlenstoffatomen, wie Methyl, Aethyl, n-Propyl, Isopropyl, n-Butyl und t-Butyl. Der Ausdruck "niederes Alkoxy" bezeichnet geradkettige oder verzweigte Alkoxygruppen mit bis zu 4 Kohlenstoffatomen, wie Methoxy, Aethoxy, n-Propoxy, Isopropoxy, n-Butoxy und t-Butoxy. Der Ausdruck "Aryl-(niederes Alkoxy)" bezeichnet eine niedere Alkoxygruppe, in welcher ein Wasserstoffatom durch die unsubstituierte Phenylgruppe oder durch eine Phenylgruppe, welche einen oder mehrere Substituenten aus der Gruppe, bestehend beispielsweise aus niederes Alkyl, niederes Alkoxy und Halogen, enthält, ersetzt ist. Beispiele für solche Aryl-niedere Alkoxygruppen sind die Benzyloxy-, die 2-Phenyläthoxygruppe und dergleichen. Methylamino, Aethoxyamino und dergleichen sind Beispiele für Mono(niedere Alkyl)aminogruppen und Dimethylamino, Diäthylamino, Aethylmethylamino und dergleichen sind Beispiele für Di(niedere Alkyl)aminogruppen. Der Ausdruck "Halogen" bedeutet Fluor, Chlor, Brom oder Jod.

Die pharmazeutisch annehmbaren Salze von Verbindungen der Formel I können Salze mit pharmazeutisch annehmbaren anorganischen Säuren, beispielsweise mit Halogenwasserstoffsäuren (z.B. Salzsäure, Bromwasserstoffsäure und Jodwasserstoffsäure), Salpetersäure und Phosphorsäure, und mit pharmazeutisch annehmbaren organischen Säuren, beispielsweise mit Essigsäure, Weinsäure, Zitronensäure, Fumarsäure, Maleinsäure, Apfelsäure, Methansulfonsäure und p-Toluolsulfonsäure sein. Diese pharmazeutisch annehmbaren Säureadditionssalze können in Form von Monosalzen oder Disalzen vorliegen. Verbindungen der Formel I, worin $R^4$ Hydroxy bedeutet, können auch pharmazeutisch annehmbare Salze mit pharmazeutisch annehmbaren Basen bilden. Beispiele solcher Salze sind die Alkalimetallsalze, wie die Natrium- und die Kaliumsalze, Eralkalimetallsalze, wie die Calciumsalze,

Ammoniumsalze und dergleichen.

Die Verbindungen der Formel I enthalten ein asymmetrisch substituiertes Kohlenstoffatom und können in racemischer oder optisch aktiver Form vorliegen. Die Formel I soll die D,L-Verbindungen, die D-Verbindungen und die L-Verbindungen umfassen.

Bevorzugte erfindungsgemässe pharmazeutische Präparate enthalten eine Verbindung der Formel I, worin $R^1$, $R^2$ und $R^3$ je Wasserstoff und $R^4$ niederes Alkoxy, insbesondere Aethoxy bedeuten, oder ein pharmazeutisch annehmbares Salz davon. Ganz besonders bevorzugte erfindungsgemässe pharmazeutische Präparate enthalten Aethyl D-α-amino-2-imidazolpropionat oder ein pharmazeutisch annehmbares Salz davon.

Beispiele für andere erfindungsgemässe pharmazeutische Präparate sind diejenigen, welche eine der folgenden Verbindungen der Formel I oder ein pharmazeutisch annehmbares Salz davon enthalten:

D,L-α-Amino-2-imidazolpropionsäure,
D-α-Amino-2-imidazolpropionsäure,
L-α-Amino-2-imidazolpropionsäure,
Methyl D,L-α-amino-2-imidazolpropionat,
Methyl L-α-amino-2-imidazolpropionat,
Methyl D-α-amino-2-imidazolpropionat,
Aethyl D,L-α-amino-2-imidazolpropionat,
Aethyl L-α-amino-2-imidazolpropionat,
Benzyl D,L-α-amino-2-imidazolpropionat,
Benzyl D-α-amino-2-imidazolpropionat,
Benzyl L-α-amino-2-imidazolpropionat,
D,L-α-Amino-α-methyl-2-imidazolpropionsäure,
D,L-α-Amino-4,5-dimethyl-2-imidazolpropionsäure,
D,L-α-Amino-2-imidazolpropionamid,
D,L-α-Amino-N-methyl-2-imidazolpropionamid und
D,L-α-Amino-N,N-dimethyl-2-imidazolpropionamid.

Die erfindungsgemässen pharmazeutischen Präparate können oral (z.B. in Form von Tabletten, Dragées, Hartgelatinekapseln, Weichgelatinekapseln, Lösungen, Emulsionen oder Suspensionen) oder parenteral (z.B. in Form von Injektionslösungen) verabreicht werden.

Als Trägermaterialien für die erfindungsgemässen pharmazeutischen Präparate eignen sich pharmazeutisch inerte, anorganische oder organische Träger. Beispiele solcher Träger, welche für Tabletten, Dragées und Hartgelatinekapseln verwendet werden können, sind Lactose, Maisstärke oder Derivate davon, Talk und Stearinsäure oder dessen Salze. Geeignete Träger für Weichgelatinekapseln sind beispielsweise pflanzliche Oele, Wachse, Fette und halbfeste und flüssige Polyole. Geeignete Träger für die Herstellung von Lösungen und Sirupen sind beispielsweise Wasser, Polyole, Saccharose, Invertzucker und Glucose. Geeignete Träger für Injektionslösungen sind z.B. Wasser, Alkohole, Polyole, Glycerin und pflanzliche Oele.

Die erfindungsgemässen pharmazeutischen Präparate können daneben noch Konservierungsmittel, Lösungsvermittler, Stabilisiermittel, Netzmittel, Emulgiermittel, Süssmittel, Farbstoffe, Aromastoffe, Salze zur Veränderung des osmotischen Druckes, Puffer, Ueberzugsmittel oder Antioxydantien enthalten. Die vorliegenden pharmazeutischen Präparate können zusätzlich zu den Verbindungen der Formel I und ihren pharmazeutisch annehmbaren Salzen noch andere therapeutisch wertvolle Substanzen enthalten.

Die erfindungsgemässen pharmazeutischen Präparate können dadurch hergestellt werden, dass man eine Verbindung der Formel I oder ein pharmazeutisch annehmbares Salz davon und gegebenenfalls eine oder mehrere andere therapeutisch wertvolle Substanzen mit einem pharmazeutischen Trägermaterial mischt und die Mischung in eine galenische Darreichungsform bringt.

Die erfindungsgemässen pharmazeutischen Präparate enthalten je nach Dosierungsform und verwendeter Verbindung der Formel I oder Salz davon etwa 125 mg bis etwa 400 mg einer Verbindung der Formel I oder eines pharmazeutisch annehmbaren Salzes davon.

Die Dosierung einer Verbindung der Formel I oder eines pharmazeutisch annehmbaren Salzes davon hängt ab vom speziellen, zu verabreichenden erfindungsgemässen Wirkstoff, der speziellen, zu behandelnden Krankheit sowie den Bedürfnissen des Patienten, wie sie vom behandelnden Arzt festgestellt werden. Die Verbindungen der Formel I und ihre pharmazeutisch annehmbaren Salze können beispielsweise zur Behandlung von degenerativen Gelenkkrankheiten in einer täglichen Dosierung von etwa 250 mg bis etwa 1500 mg und zur Behandlung der Wilson-Krankheit in einer täglichen Dosierung bis zu 3 g verabreicht werden. Es ist klar, dass diese Dosierungen nur als Beispiele zu verstehen sind und nach oben oder nach unten variieren können.

Die pharmakologische Wirkung der Verbindungen der Formel I kann in den folgenden Tests gezeigt werden:

(i) <u>In-vitro-Test zur Bestimmung von Superoxid-Dismutase-ähnlicher Wirkung und der Stabilität von Kupferchelaten von Verbindungen der Formel I</u>:

Man stellt $10^{-2}$M-Lösungen her, welche Kupfer in Form von Kupfer(II)bisglycinat und Testverbindung enthalten, mischt diese in einem molaren Verhältnis von 1:4 (Kupferverbindung:Testsubstanz) und verdünnt mit 100 mM-Hepes-Puffer von pH 7,8, so dass die Kupferkonzentration $10^{-3}$M/l beträgt. Man verdünnt diese Lösung mit obigem Puffer 10-fach, vermischt sie in verschiedenen bekannten Verhältnissen mit Aethylendiamin-tetraessigsäure und lässt dann die erhaltenen Lösungen zum Aequilibrieren bei Raumtemperatur während 1 Stunde stehen. Man entnimmt diesen Lösungen Aliquote, welche eine berechnete Aethylendiamin-tetraessig-

säure-Konzentration von 20 µM/l besitzen, und testet diese wie folgt auf ihre Fähigkeit hin, die Reduktion von Nitroblautetrazolium durch das Superoxidradikal zu hemmen.

Die Versuchslösung enthält $2 \times 10^{-5}$M Hypoxanthin, $5 \times 10^{-4}$M Nitroblautetrazolium, 1 mg/ml Gelatine und 100 mM Hepes-Puffer vom pH 7,8 und hat ein Gesamtvolumen von 3,0 ml. Man startet die Reaktion durch Zugabe von 30 µl einer Xanthinoxidase-Lösung (0,1 Einheiten/ml) und lässt dann bis zur Beendigung der Reaktion bei Raumtemperatur stehen (gewöhnlich über Nacht). Die Reduktion von Nitroblautetrazolium wird photometrisch bei 550 nm gemessen.

Bei Anwesenheit einer Superoxid-Dismutase-ähnlichen Wirkung wird die Reduktion von Nitroblautetrazolium gehemmt, und die für eine 50-proz. Hemmung benötigte Konzentration an Kupfer ($I_{50}$) kann unter Einhaltung einer Aethylendiamin-tetraessigsäure-Konzentration von 20 µM bestimmt werden.

Die im obigen Test erhaltenen Resultate sind in der Tabelle I zusammengestellt:

Tabelle I

| Testverbindung | $I_{50}$ µM |
|---|---|
| D,L-α-Amino-2-imidazolpropionsäure | 3,5 |
| L-α-Amino-2-imidazolpropionsäure | 3,5 |
| D-α-Amino-2-imidazolpropionsäure | 3,5 |
| Methyl D,L-α-amino-2-imidazolpropionat | 2,5 |
| Methyl D-α-amino-2-imidazolpropionat | 3,0 |
| Methyl L-α-amino-2-imidazolpropionat | 3,0 |
| Aethyl D,L-α-amino-2-imidazolpropionat | 2,5 |
| Aethyl D-α-Amino-2-imidazolpropionat | 2,5 |
| Aethyl L-α-amino-2-imidazolpropionat | 2,5 |
| Benzyl D,L-α-amino-2-imidazolpropionat | 2,0 |
| Benzyl D-α-amino-2-imidazolpropionat | 2,0 |
| Benzyl L-α-amino-2-imidazolpropionat | 2,0 |
| D,L-α-Amino-α-methyl-2-imidazolpropionsäure | 3,0 |
| D,L-α-Amino-4,5-dimethyl-2-imidazolpropionsäure | 8,5 |
| D,L-α-Amino-N-methyl-2-imidazolpropionamid | 3,5 |
| D,L-α-Amino-2-imidazolpropionamid | 3,0 |
| D,L-α-Amino-N,N-dimethyl-2-imidazolpropionamid | 3,5 |

(ii) In-vivo-Cupriorese-Test:

Die Testmethode basiert auf derjenigen von K. Gibbs und J.M. Walshe (Clin. Sci. Mol. Med. 53, 317-320, 1977) und wurde wie folgt durchgeführt:

Man lässt männliche, 120-200 g schwere Ratten am Morgen des Versuchstages hungern. Am späten Nachmittag des Versuchstages verabreicht man den Ratten in Vierergruppen die Testsubstanz oral (10 ml/kg) und bringt sie einzeln in Stoffwechselkäfige, wo sie während 18 Stunden bleiben. Während dieser Zeit haben die Ratten Zugang zu deionisiertem Wasser ad libitum und zu einer 10 g schweren Futterpille, bestehend aus pulverisiertem Rattenfutter, das mit Erdnussöl und Tragacanth plus Tween 80 in destilliertem Wasser gebunden ist. Man sammelt in dieser Zeit den ausgeschiedenen Urin getrennt vom Kot, bestimmt für jede Ratte

das Volumen des ausgeschiedenen Urins und bestimmt die Kupferkonzentration mittels Absorptionsspektrophotometrie bei 324 nm. Man drückt die Kupferkonzentration in µMol Cu/kg aus und berechnet die prozentuale Veränderung im Vergleich zu Kontrolltieren mittels der folgenden Formel:

$$\frac{(\text{Testwert-Kontrollwert})}{\text{Kontrollwert}} \times 100\%.$$

Die im obigen Test erhaltenen Resultate sind in der folgenden Tabelle II zusammengestellt:

Tabelle II

| Testsubstanz | Cupriuretische Wirkung | |
|---|---|---|
| | Dosierung mMol/kg p.o. | prozentuale Aenderung im Vergleich zu Kontrolltieren |
| D,L-α-Amino-2-imidazolpropionsäure | 1,0 | +345 |
| D-α-Amino-2-imidazolpropionsäure | 1,0 | +143 |
| L-α-Amino-2-imidazolpropionsäure | 1,0 | +475 |
| L-α-Amino-2-imidazolpropionsäure | 0,3 | +273 |
| Methyl D,L-α-amino-2-imidazolpropionat | 1,0 | +241 |
| Methyl D-α-amino-2-imidazolpropionat | 1,0 | +277 |
| Methyl L-α-amino-2-imidazolpropionat | 0,3 | +237 |
| Aethyl D,L-α-amino-2-imidazolpropionat | 0,3 | +115 |
| Aethyl D-α-amino-2-imidazolpropionat | 1,0 | +266 |
| Aethyl D-α-amino-2-imidazolpropionat | 0,3 | +131 |
| Aethyl L-α-amino-2-imidazolpropionat | 0,3 | +154 |
| Benzyl D,L-α-amino-2-imidazolpropionat | 0,3 | +130 |
| Benzyl D-α-amino-2-imidazolpropionat | 1,0 | +185 |
| | 0,3 | + 56 |
| Benzyl L-α-amino-2-imidazolpropionat | 1,0 | +302 |
| | 0,3 | +176 |
| D,L-α-Amino-α-methyl-2-imidazolpropionsäure | 1,0 | + 55 |
| D,L-α-Amino-4,5-dimethyl-2-imidazolpropionsäure | 1,0 | + 12 |

| | | |
|---|---|---|
| D,L-α-Amino-2-imidazolpropionamid | 1,0 | +184 |
| D,L-α-Amino-N-methyl-2-imidazolpropionamid | 1,0 | +168 |
| D,L-α-Amino-N,N-dimethyl-2-imidazolpropionamid | 1,0 | +200 |

Mit Ausnahme der D,L-Verbindungen der Formel I, worin $R^1$, $R^2$ und $R^3$ je Wasserstoff und $R^4$ Hydroxy oder Methoxy bedeuten, sind die Verbindungen der Formel I neu. Diese neuen Verbindungen der Formel I und ihre pharmazeutisch annehmbaren Salze sowie ein Verfahren zu deren Herstellung sind ebenfalls Gegenstand der vorliegenden Erfindung.

In einer speziellen Ausführungsform betrifft die vorliegende Erfindung Verbindungen der Formel I, worin $R^4$ eine von Hydroxy oder Methoxy verschiedene Bedeutung hat, wenn $R^1$, $R^2$ und $R^3$ je Wasserstoff bedeuten.

Bevorzugte neue Verbindungen der Formel I sind diejenigen, worin $R^1$, $R^2$ und $R^3$ je Wasserstoff und $R^4$ niederes Alkoxy mit mindestens 2 Kohlenstoffatomen bedeuten. Eine besonders bevorzugte neue Verbindung der Formel I ist das Aethyl D-α-amino-2-imidazolpropionat.

Die neuen Verbindungen der Formel I und ihre pharmazeutisch annehmbaren Salze können erfindungsgemäss hergestellt werden, indem man

a) zur Herstellung einer D,L-Verbindung der Formel I, worin mindestens eines von $R^1$, $R^2$ und $R^3$ niederes Alkyl und $R^4$ Hydroxy bedeuten, eine D,L-Verbindung der allgemeinen Formel

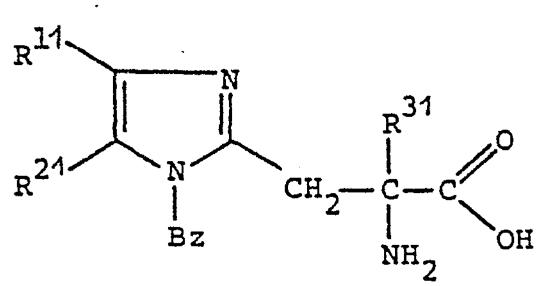

II

worin $R^{11}$, $R^{21}$ und $R^{31}$ je Wasserstoff oder niederes Alkyl und Bz Benzyl bedeuten, wobei mindestens eines von $R^{11}$, $R^{21}$ und $R^{31}$ niederes Alkyl bedeutet,

debenzyliert, oder

b) zur Herstellung einer D,L-, D- oder L-Verbindung der Formel I, worin $R^4$ niederes Alkoxy mit mindestens 2 Kohlenstoffatomen bedeutet, oder einer D- oder L-Verbindung der Formel I, worin $R^4$ Methoxy bedeutet, oder einer D,L-Verbindung der Formel I, worin $R^4$ Aryl-niederes Alkoxy bedeutet, eine entsprechende D,L-, D- oder L-Verbindung der Formel I, worin $R^4$ Hydroxy bedeutet, verestert, oder

c) zur Herstellung einer D,L-, D- oder L-Verbindung der Formel I, worin $R^4$ Amino, Mono(niederes Alkyl)amino oder Di(niederes alkyl)amino bedeutet, eine D,L-, D- oder L-Verbindung der Formel I, worin $R^4$ Hydroxy oder niederes Alkoxy bedeutet, entsprechend amidiert, oder

d) zur Herstellung einer D,L-, D- oder L-Verbindung der Formel I, worin $R^4$ niederes Alkoxy mit mindestens 2 Kohlenstoffatomen oder Aryl-niederes Alkoxy bedeutet, oder einer D- oder L-Verbindung der Formel I, worin $R^4$ Methoxy bedeutet, eine D,L-, D- oder L-Verbindung der allgemeinen Formel

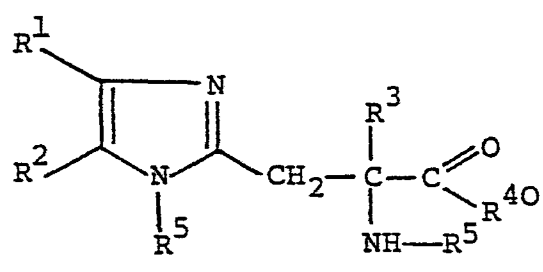

IIIa

worin $R^1$, $R^2$ und $R^3$ obige Bedeutung besitzen, $R^{40}$ niederes Alkoxy oder Aryl-niederes Alkoxy und $R^5$ t-Butoxycarbonyl bedeuten,

hydrolysiert, oder

e) zur Herstellung einer D- oder L-Verbindung der Formel I, worin $R^4$ Hydroxy bedeutet, oder einer D,L-Verbindung der Formel I, worin mindestens eines von $R^1$, $R^2$ und $R^3$ niederes Alkyl und $R^4$ Hydroxy bedeuten, eine entsprechende D-, L- oder D,L-Verbindung der Formel I, worin $R^4$ niederes Alkoxy bedeutet, hydrolysiert, oder

f) zur Herstellung einer D- oder L-Verbindung der Formel I, eine D,L-Verbindung der Formel I einer Racematspaltung unterwirft, und die D- oder L-Verbindung isoliert, und erwünschtenfalls eine erhaltene Verbindung der Formel I in ein pharmazeutisch annehmbares Salz überführt.

Die Debenzylierung einer Verbindung der Formel II gemäss Verfahren a) kann in an sich bekannter Weise durchgeführt werden. Zweckmässigerweise wird die Debenzylierung durch Behandeln mit Natrium in flüssigem Ammoniak durchgeführt.

Die Veresterung gemäss Verfahren b) kann in an sich bekannter Weise durchgeführt werden. Man kann die Veresterung beispielsweise dadurch bewerkstelligen, dass man eine Verbindung der Formel I, worin $R^4$ Hydroxy bedeutet, mit einem niederen Alkanol, wie Methanol und Aethanol, oder einem Aryl substituierten niederen Alkanol, wie z.B. Benzylalkohol, in Gegenwart einer starken Säure, beispielsweise einer Halogenwasserstoffsäure, wie z.B. Salzsäure, oder einer Sulfonsäure, wie z.B. p-Toluolsulfonsäure, umsetzt.

Die Amidierung gemäss Verfahren c) kann ebenfalls in an sich bekannter Weise durchgeführt werden. In einer bevorzugten Ausführungsform wird die Amidierung durch Reaktion einer Verbindung der Formel I, worin $R^4$ niederes Alkoxy bedeutet, mit Ammoniak, einem niederen Alkylamin (z.B. Methylamin) oder einem Di(niederen alkyl)amin (z.B. Dimethylamin) bewerkstelligt, wobei man zweckmässigerweise ein inertes organisches Lösungsmittel verwendet (z.B. einen niederen Alkohol, wie Methanol, etc.).

Die Hydrolyse einer Verbindung der Formel III gemäss Verfahren d) kann unter Verwendung von Chlorwasserstoff in Dioxan in an sich bekannter Weise durchgeführt werden.

Die Hydrolyse gemäss Verfahren e) kann unter Verwendung von Salzsäure ebenfalls in an sich bekannter Weise durchgeführt werden.

Die Racematspaltung gemäss Verfahren f) kann in an sich bekannter Weise durchgeführt werden. Man kann beispielsweise eine D,L-Verbindung der Formel I mit einer optisch aktiven Säure, wie D-Weinsäure, L-Weinsäure, Dibenzoyl-D-weinsäure und Dibenzoyl-L-weinsäure, umsetzen, die beiden erhaltenen diastereoisomeren Salze auftrennen (beispielsweise durch fraktionierte Kristallisation) und aus den erhaltenen Salzen die optisch einheitlichen Verbindungen der Formel I freisetzen.

Die Ueberführung einer erhaltenen neuen Verbindung der Formel I in ein pharmazeutisch annehmbares Salz kann durch Behandeln mit einer pharmazeutisch annehmbaren Säure oder einer pharmazeutisch annehmbaren Base in an sich bekannter Weise bewerkstelligt werden.

Die Ausgangsstoffe der obigen Formel II sind entweder bekannt oder Analoga von bekannten Verbindungen, welche in Analogie zu den bekannten Verbindungen hergestellt werden können.

Die Ausgangsstoffe der Formel IIIa sind neu und ebenfalls Gegenstand der vorliegenden Erfindung. Sie können hergestellt werden, indem man eine D,L-, D- oder L-Verbindung der Formel I, worin $R^4$ Hydroxy bedeutet, mit Di-t-butyldicarbonat umsetzt und die erhaltenen D,L-, D- oder L-Verbindung der allgemeinen Formel

0118787

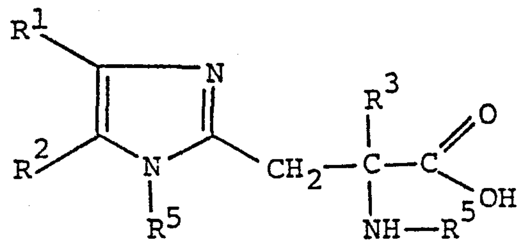

IIIb

worin $R^1$, $R^2$, $R^3$ und $R^5$ obige Bedeutung besitzen, verestert.

Die Reaktion einer Verbindung der Formel I, worin $R^4$ Hydroxy bedeutet, mit Di-t-butyldicarbonat kann in Gegenwart einer Base (beispielsweise eines Alkalimetallhydroxides, wie Natriumhydroxid oder Kaliumhydroxid) in einem inerten organischen Lösungsmittel (beispielsweise einem cyclischen Aether, wie Dioxan) zweckmässigerweise bei etwa 0°C in an sich bekannter Weise durchgeführt werden.

Die Veresterung einer Verbindung der Formel IIIb kann ebenfalls in an sich bekannter Weise durchgeführt werden, und zwar unter Verwendung eines geeigneten Diazoalkans (z.B. von Diazomethan) oder eines geeigneten Aryl-diazoalkans (z.B. von Phenyldiazomethan), zweckmässigerweise in einem inerten organischen Lösungsmittel (z.B. in einem Aether, wie Diäthyläther) und bei etwa Raumtemperatur.

Die Verbindungen der Formel IIIb sind neu und ebenfalls Gegenstand der vorliegenden Erfindung.

Gegenstand der vorliegenden Erfindung sind demnach: Pharmazeutische Präparate, enthaltend eine Verbindung der Formel I; ein Verfahren zur Herstellung solcher pharmazeutischer Präparate; die Verbindungen der Formel I als pharmazeutische Wirkstoffe und, mit Ausnahme der D,L-Verbindungen der Formel I, worin $R^1$, $R^2$ und $R^3$ je Wasserstoff und $R^4$ Hydroxy oder Methoxy bedeuten, auch per se; ein Verfahren zur Herstellung der neuen Verbindungen der Formel I; die Verwendung von Verbindungen der Formel I und von Präparaten, enthaltend dieselben, bei der Behandlung

von degenerativen Gelenkkrankheiten und der Wilson-Krankheit; sowie Zwischenprodukte für die Herstellung der neuen Verbindungen der Formel I.

Die nachfolgenden Beispiele erläutern die vorliegende Erfindung.

## I. Pharmazeutische Präparate

### Beispiel 1

Tabletten, enthaltend die folgenden Bestandteile, können in herkömmlicher Weise hergestellt werden:

| | | |
|---|---|---|
| Aethyl D-α-amino-2-imidazolpropionat | | 400 mg |
| Mikrokristalline Zellulose | | 70 mg |
| Kalziumphosphat-dihydrat | | 136 mg |
| Natriumstärke-glycolat | | 30 mg |
| Polyvinylpyrrolidon | | 20 mg |
| Talk | | 30 mg |
| Magnesiumstearat | | 4 mg |
| | Gesamtgewicht | 690 mg |

### Beispiel 2

Hartgelatinekapseln, enthaltend die folgenden Bestandteile, können in herkömmlicher Weise hergestellt werden:

| | | |
|---|---|---|
| Aethyl D-α-amino-2-imidazolpropionat | | 200 mg |
| Mannitol | | 150 mg |
| Stärke | | 72 mg |
| Natrium-dioctylsulphosuccinat | | 1 mg |
| Talk | | 15 mg |
| Magnesiumstearat | | 2 mg |
| | Kapselfüllgewicht | 440 mg |

### Beispiel 3

Ein Sirup, enthaltend die folgenden Bestandteile, kann in herkömmlicher Weise hergestellt werden:

| | | | |
|---|---|---|---|
| Aethyl D-α-amino-2-imidazolpropionat | | 200 | mg |
| Sorbitol (70-proz. wässrige Lösung) | | 2 | mg |
| Zitronensäure | | 1,5 | mg |
| Saccharin | | 10 | mg |
| Aromastoffe | | 10 | mg |
| Farbstoffe | | 1 | mg |
| Methyl p-hydroxybenzoat | | 5 | mg |
| Propyl p-hydroxybenzoat | | 1 | mg |
| destilliertes Wasser | ad | 5 | ml |

Beispiel 4

Eine feste Form für die Zubereitung eines Sirups, enthaltend die folgenden Bestandteile, kann in herkömmlicher Weise hergestellt werden:

| | | |
|---|---|---|
| Aethyl D-α-amino-2-imidazolpropionat | 200 | mg |
| Sucrose | 1,8 | mg |
| Zitronensäure | 2 | mg |
| Natriumcyclamat | 10 | mg |
| Natriumsaccharinat | 5 | mg |
| Farbstoffe | 1 | mg |
| Aromastoffe | 4 | mg |
| Hydroxypropylmethylzellulose | 5 | mg |

Diese feste Form wird vor der Verwendung mit soviel Wasser zubereitet, dass man 5 ml eines wohlschmeckenden Sirups erhält.

Beispiel 5

Tabletten, enthaltend die folgenden Bestandteile, können in herkömmlicher Weise hergestellt werden:

| | |
|---|---|
| D,L-α-Amino-2-imidazolpropionsäure | 250 mg |
| Mikrokristalline Zellulose | 130 mg |
| Kalziumphosphat-dihydrat | 170 mg |
| Natriumstärke-glycolat | 30 mg |
| Talk | 37 mg |
| Magnesiumstearat | 3 mg |
| Gesamtgewicht | 620 mg |

## Beispiel 6

Tabletten, enthaltend die folgenden Bestandteile, können in herkömmlicher Weise hergestellt werden:

| | |
|---|---|
| D,L-α-Amino-2-imidazolpropionsäure | 125 mg |
| Mannitol | 70 mg |
| Mikrokristalline Zellulose | 70 mg |
| Natriumstärke-glycolat | 20 mg |
| Hydroxypropylmethylzellulose | 8 mg |
| Talk | 15 mg |
| Magnesiumstearat | 2 mg |
| Gesamtgewicht | 310 mg |

## Beispiel 7

Hartgelatinekapseln, enthaltend die folgenden Bestandteile, können in herkömmlicher Weise hergestellt werden:

| | |
|---|---|
| D,L-α-Amino-2-imidazolpropionsäure | 200 mg |
| Mannitol | 150 mg |
| Stärke | 72 mg |
| Natrium-dioctylsulphosuccinat | 1 mg |
| Talk | 15 mg |
| Magnesiumstearat | 2 mg |
| Gesamtgewicht | 440 mg |

## Beispiel 8

Ein Sirup, enthaltend die folgenden Bestandteile, kann in herkömmlicher Weise hergestellt werden:

| | | | |
|---|---|---|---|
| D,L-α-Amino-2-imidazolpropionsäure | | 200 | mg |
| Sorbitol (70-proz. wässrige Lösung) | | 2 | g |
| Zitronensäure | | 1,5 | mg |
| Saccharin | | 10 | mg |
| Aromastoffe | | 10 | mg |
| Farbstoffe | | 1 | mg |
| Methyl p-hydroxybenzoat | | 5 | mg |
| Propyl p-hydroxybenzoat | | 1 | mg |
| Destilliertes Wasser | ad | 5 | ml |

## Beispiel 9

Eine feste Form, enthaltend die folgenden Bestandteile, kann in herkömmlicher Weise hergestellt werden:

| | | |
|---|---|---|
| D,L-α-Amino-2-imidazolpropionsäure | 200 | mg |
| Sucrose | 1,8 | g |
| Zitronensäure | 2 | mg |
| Natriumcyclamat | 10 | mg |
| Natriumsaccharinat | 5 | mg |
| Farbstoffe | 1 | mg |
| Aromastoffe | 4 | mg |
| Hydroxypropylmethylcellulose | 5 | mg |

Diese feste Form wird vor der Verwendung mit soviel Wasser zubereitet, dass man 5 ml eines wohlschmeckenden Sirups erhält.

II. Herstellung von neuen Verbindungen der Formel I und ihren pharmazeutisch annehmbaren Salzen:

## Beispiel 10

Man suspendiert 3,1 g L-α-Amino-2-imidazolpropionsäure in 60 ml Methanol und leitet unter Rühren und Kühlen mit Eis trockenen Chlorwasserstoff in die Suspension ein, wobei man bald eine klare Lösung erhält. Man fährt mit dem Gaseinleiten fort, bis die Lösung gesättigt ist (1,25 Stunden). Man lässt die erhaltene Lösung über Nacht bei Raumtemperatur stehen, dampft zur Trockene ein, nimmt den Rückstand in 10 ml Methanol auf und verdünnt die erhaltene Lösung mit 100 ml trockenem Diäthyläther. Das ausgefallene Sirupartige Material wird nach Animpfen zu einem weissen Festkörper, den man abfiltriert, mit Diäthyläther wäscht und trocknet. Man erhält 4,79 g eines Produktes mit einem Schmelzpunkt von 189°C (Schaumbildung), $[\alpha]_D^{20}$ = +43,4° (c = 0,99% in 0,1N Salzsäure). Man löst dieses Material durch Erwärmen in 50 ml Methanol/Essigester (1:1), filtriert die heisse Lösung und versetzt das siedende Filtrat mit Essigester bis zur beginnenden Kristallisation, wobei man 15 ml Essigester benötigt . Man lässt die Mischung auf Raumtemperatur abkühlen und lässt sie dann über Nacht in einem Kühlschrank stehen. Man erhält 3,3 g Methyl L-α-amino-2-imidazolpropionat-dihydrochlorid vom Schmelzpunkt 189°C (Schaumbildung), $[\alpha]_D^{20}$ = +43,7° (c = 1,05% in 0,1N-Salzsäure).

Analyse für $C_7H_{11}N_3O_2.2HCl$:

| | |
|---|---|
| Berechnet: | C 34,7; H 5,4; N 17,4; Cl 29,3%. |
| Gefunden: | C 34,6; H 5,4; N 17,5; Cl 29,5%. |

## Beispiel 11

In Analogie zu Beispiel 10 erhält man aus 3,1 g D-α-Amino-2-imidazolpropionsäure 3,1 g Methyl D-α-amino-2-imidazolpropionat-dihydrochlorid vom Schmelzpunkt 189°C (Schaumbildung), $[\alpha]_D^{20}$ = -43,3° (c = 0,97% in 0,1N-Salzsäure).

Analyse für $C_7H_{11}N_3O_2 \cdot 2HCl$:
Berechnet: C 34,7; H 5,4; N 17,4; Cl 29,3%.
Gefunden: C 34,6; H 5,4; N 17,4; Cl 29,4%.

## Beispiel 12

Durch eine gerührte und mit Eis gekühlte Suspension von 5 g (32 mMol) D,L-α-Amino-2-imidazolpropionsäure in 100 ml Aethanol leitet man trockenes Chlorwasserstoffgas, und zwar bis die Mischung gesättigt ist. Man rührt die Mischung während 75 Stunden bei Raumtemperatur, dampft die erhaltene klare Lösung zur Trockene ein, nimmt den Rückstand in 5 ml Wasser auf und verdünnt die Lösung mit 100 ml Acetonitril. Das ausgefallene Sirup-artige Material kristallisiert langsam zu einem weissen Festkörper, der abfiltriert, mit Acetonitril gewaschen und bei Raumtemperatur an der Luft über Nacht getrocknet wird. Man erhält 9,8 g eines Produktes mit einem Schmelzpunkt von 45-48°C. Nach Umkristallisieren aus 5 ml Wasser erhält man 3,0 g Aethyl D,L-α-amino-2-imidazolpropionat-dihydrochlorid-trihydrat in Form farbloser Kristalle vom Schmelzpunkt 46-48,5°C.
Analyse für $C_8H_{13}N_3O_2 \cdot 2HCl \cdot 3H_2O$:
Berechnet: C 31,0; H 6,8; N 13,6; $H_2O$ 17,4%.
Gefunden: C 31,0; H 6,6; N 13,6; $H_2O$ 17,2%.

## Beispiel 13

In Analogie zu Beispiel 12 erhält man aus 2,0 g (13 mMol) D-α-Amino-2-imidazolpropionsäure nach Umkristallisieren aus Wasser 1,4 g Aethyl D-α-amino-2-imidazolpropionat-dihydrochlorid-trihydrat vom Schmelzpunkt 59-61°C, $[\alpha]_D^{20}$ = -40,3° (c = 1,00% in Wasser).
Analyse für $C_8H_{13}N_3O_2 \cdot 2HCl \cdot 3H_2O$:
Berechnet: C 31,0; H 6,8; N 13,6; $H_2O$ 17,4%.
Gefunden: C 30,7; H 6,6; N 13,6; $H_2O$ 18,0%.

Beispiel 14

In Analogie zu Beispiel 12 erhält man aus 2,0 g (13 mMol) L-α-Amino-2-imidazolpropionsäure nach Umkristallisieren aus Wasser 1,0 g Aethyl L-α-amino-2-imidazolpropionat-dihydrochlorid-trihydrat vom Schmelzpunkt 59-61°C, $[\alpha]_D^{20}$ = +40,3° (c = 1,01% in Wasser).

Analyse für $C_8H_{13}N_3O_2.2HCl.3H_2O$:

Berechnet: C 31,0; H 6,8; N 13,6; $H_2O$ 17,4%.

Gefunden: C 31,0; H 6,6; N 13,7; $H_2O$ 17,6%.

Beispiel 15

Man suspendiert 68 g (0,22 Mol) Aethyl D,L-α-amino-2-imidazolpropionat-dihydrochlorid-trihydrat in 680 ml Aethanol, versetzt die Suspension unter Rühren mit einer Lösung von 10,1 g (0,44 g Atom) Natrium in 200 ml Aethanol, rührt die Mischung während 30 Minuten und dampft dann zur Trockene ein. Man behandelt den Rückstand mit 1 l Chloroform und filtriert durch Hyflo, um Natriumchlorid zu entfernen. Nach Eindampfen des Filtrates erhält man 41,2 g kristallines Aethyl D,L-α-amino-2-imidazolpropionat. Man nimmt dieses Material in 590 ml Aethanol auf und versetzt mit einer Lösung von 84,7 g (0,22 Mol) (+)-Dibenzoyl-D-weinsäure-monohydrat in 590 ml Aethanol. Man lässt die erhaltene Lösung während 20 Stunden in einem Kühlschrank stehen. Die gebildete harte Kristallmasse wird zerkleinert und die Mischung wird für weitere 24 Stunden im Kühlschrank stehen gelassen. Das Produkt wird abfiltriert, zweimal mit je 50 ml Aethanol gewaschen und zu konstantem Gewicht getrocknet. Man erhält 60,1 g rohes Dibenzoyl-D-tartrat vom Schmelzpunkt 140-141,5°C (Zersetzung), $[\alpha]_D^{20}$ = +69,8° (c = 0,99% in Methanol). Durch Umkristallisieren dieses Produktes aus 1,1 l Aethanol, das 20 ml Wasser enthält, erhält man 44,6 g Material vom Schmelzpunkt 143-145°C (Zersetzung), $[\alpha]_D^{20}$ = +67,6° (c = 1,00% in Methanol). Schliesslich wird noch aus 1,5 l Aethanol, das 4 ml Wasser enthält, umkristallisiert, worauf man 32,8 g Aethyl D-α-

amino-2-imidazolpropionat-dibenzoyl-D-tartrat vom Schmelzpunkt 141-145°C (Zersetzung) erhält, $[\alpha]_D^{20}$ = +65,9° (c = 1,00% in Methanol).

Analyse für $C_{26}H_{27}N_3O_{10}.1,1H_2O$:

Berechnet: C 55,6; H 5,2; N 7,5; $H_2O$ 3,5%.

Gefunden: C 55,4; H 5,2; N 7,4; $H_2O$ 3,6%.

Man suspendiert 31,8 g (0,057 Mol) Aethyl D-α-amino-2-imidazolpropionat-dibenzoyl-D-tartrat in 400 ml Aethanol, versetzt unter Rühren mit einer Lösung von Chlorwasserstoff in Aethanol (32 ml, ca. 5,6M) und dampft die erhaltene klare Lösung zur Trockene ein. Der erhaltene Sirup wird mit 18 ml Wasser solange geschüttelt, bis man eine homogene Mischung erhält. Diese Mischung wird mit 360 ml Acetonitril erwärmt und dann abgekühlt. Die ausgeschiedenen Kristalle werden abfiltriert, mit Acetonitril gewaschen und an der Luft über Nacht getrocknet. Man erhält 16,4 g eines Produktes vom Schmelzpunkt 58,5-61°C. Durch Umkristallisieren aus 8 ml Wasser erhält man 8,3 g Aethyl D-α-amino-2-imidazolpropionat-dihydrochlorid-trihydrat vom Schmelzpunkt 59-61°C, $[\alpha]_D^{20}$ = -41,4° (c = 0,99% in Wasser).

Analyse für $C_8H_{13}N_3O_2.2HCl.3H_2O$:

Berechnet: C 31,0; H 6,8; N 13,55; $H_2O$ 17,4%.

Gefunden: C 31,1; H 6,8; N 13,5; $H_2O$ 17,3%.

Die nach Abfiltrieren der ersten Portion an Aethyl D-α-amino-2-imidazolpropionat-dibenzoyl-D-tartrat erhaltenen Mutterlaugen liefern beim Stehenlassen noch eine zweite Portion eines Festkörpers. Dieser wird abfiltriert und getrocknet, wobei man 11,2 g eines Material vom Schmelzpunkt 140-142°C (Zersetzung) erhält, $[\alpha]_D^{20}$ = +77,1° (c = 1,01% in Methanol). Man behandelt das Filtrat mit einer Lösung von Chlorwasserstoff in Aethanol (100 ml, ca. 4M) und dampft dann zur Trockene ein. Man schüttelt den Rückstand mit 31 ml Wasser bis man eine homogene Mischung erhält. Diese Mischung wird mit 620 ml Acetonitril behandelt, worauf man im Kühlschrank stehen lässt. Das gebildete kristalline Material

wird abfiltriert, mit Acetonitril gewaschen und während 24 Stunden an der Luft getrocknet. Man erhält 24,2 g Material vom Schmelzpunkt 59-61°C. Durch Umkristallisieren aus 16 ml Wasser erhält man 9,0 g Aethyl L-α-amino-2-imidazolpropionat-dihydrochlorid-trihydrat vom Schmelzpunkt 58,5-60,5°C, $[\alpha]_D^{20}$ = +41,2° (c = 1,05% in Wasser).

Analyse für $C_8H_{13}N_3O_2.2HCl.3H_2O$:

Berechnet: C 31,0; H 6,8; N 13,55; $H_2O$ 17,4%.

Gefunden: C 31,1; H 6,65; N 13,6; $H_2O$ 17,4%.

## Beispiel 16

40 g (0,22 Mol) Aethyl D,L-α-amino-2-imidazolpropionat werden wie in Beispiel 15 beschrieben, jedoch unter Verwendung von 82,2 g (0,22 Mol) (-)-Dibenzoyl-L-weinsäure als Spaltungsmittel, aufgespalten. Das erhaltene rohe Salz (49,8 g) wird einmal aus 1 l Aethanol, das 3 ml Wasser enthält, umkristallisiert und liefert 39,5 g Aethyl L-α-amino-2-imidazolpropionat-dibenzoyl-L-tartrat vom Schmelzpunkt 145,5-148°C, $[\alpha]_D^{20}$ = -65,4° (c = 1,00% in Methanol).

Das obige Aethyl L-α-amino-2-imidazolpropionat-dibenzoyl-L-tartrat wird in Analogie zu Beispiel 15 in das Aethyl L-α-amino-imidazolpropionat-dihydrochlorid-trihydrat übergeführt. Man erhält nach Umkristallisieren aus Wasser 10,8 g des gewünschten Produktes vom Schmelzpunkt 58,5-60,5°C, $[\alpha]_D^{20}$ = +41,0° (c = 1,01% in Wasser).

Analyse für $C_8H_{13}N_3O_2.2HCl.3H_2O$:

Berechnet: C 31,0; H 6,8; N 13,55; $H_2O$ 17,4%.

Gefunden: C 31,15; H 6,8; N 13,7; $H_2O$ 17,7%.

Aus den Mutterlaugen dieser Racematspaltung konnte kein optisch reines Aethyl D-α-amino-2-imidazolpropionat isoliert werden. Das mit dem D-Enantiomeren angereicherte Ester-dihydrochlorid wird in die freie Base übergeführt, und diese wird mit (+)-Dibenzoyl-D-weinsäure behandelt. Das erhaltene Dibenzoyl-D-tartrat wird bis zur konstanten Rotation aus Aethanol/Wasser umkristallisiert und dann wie

in Beispiel 15 beschrieben in das Dihydrochlorid übergeführt. Durch Umkristallisieren aus Wasser erhält man 8,3 g reines Aethyl D-α-amino-2-imidazolpropionat-dihydrochlorid-trihydrat vom Schmelzpunkt 59-61°C, $[\alpha]_D^{20}$ = -41,4° (c = 0,99% in Wasser).

Analyse für $C_8H_{13}N_3O_2.2HCl.3H_2O$:

Berechnet: C 31,0; H 6,8; N 13,55; $H_2O$ 17,4%.

Gefunden: C 31,1; H 6,8; N 13,5 ; $H_2O$ 17,3%.

## Beispiel 17

Man löst 34,5 g (0,19 Mol) Aethyl D-α-amino-2-imidazolpropionat in 350 ml Aethanol und versetzt mit 185 ml einer 1N-Standard-Salzsäurelösung. Die erhaltene Lösung wird zur Trockene eingedampft, worauf man den Rückstand mit Aethanol versetzt und erneut eindampft, um vorhandenes Wasser zu entfernen. Das rohe Monohydrochlorid wird in 140 ml 95-proz. Aethanol gelöst, und die erhaltene Lösung wird mit 140 ml Essigester verdünnt. Die Mischung wird zum Sieden erhitzt und dann filtriert, worauf man das Filtrat abkühlen und dann in einem Kühlschrank über Nacht stehen lässt. Der kristallien Festkörper wird abfiltriert, mit Essigester gewaschen und während 24 Stunden an der Luft getrocknet. Man erhält 32,7 g Aethyl D-α-amino-2-imidazolpropionat-monohydrochlorid-hemihydrat vom Schmelzpunkt 98,5-100,5°C, $[\alpha]_D^{20}$ = -54,8° (c = 1,00% in 1N-Salzsäure).

Analyse für $C_8H_{13}N_3O_2.HCl.0,5H_2O$:

Berechnet: C 42,0; H 6,6; N 18,4; Cl 15,5; $H_2O$ 3,9%.

Gefunden: C 41,8; H 6,6; N 18,5; Cl 15,45; $H_2O$ 4,5%.

## Beispiel 18

Eine Lösung von 10 g Aethyl D-α-amino-2-imidazolpropionat-dihydrochlorid-trihydrat in 100 ml 6N-Salzsäure wird während 2 Stunden unter Rückfluss zum Sieden erhitzt. Die Mischung wird dann bei 40°C unter vermindertem Druck zur Trockene eingedampft, worauf man den Rückstand zweimal zusammen mit je 40 ml Wasser eindampft, um die Salzsäure

grösstenteils zu entfernen. Man nimmt den Rückstand anschliessend in 50 ml Wasser auf und gibt die Lösung auf eine Kolonne, welche 150 g eines Kationenaustauscherharzes enthält (Zerolit 225, $H^+$-Form). Die Säule wird dann gründlich mit Wasser gewaschen, und zwar solange bis das Eluat neutral ist. Die D-α-Amino-2-imidazolpropionsäure wird dann mit 1N-Ammoniak eluiert. Die gesammelten Fraktionen, welche mit Ninhydrin positiv reagieren, werden vereinigt und zur Trockene eingedampft, wobei man 4,7 g eines weissen Festkörpers erhält. Zum Umkristallisieren wird dieser Festkörper in 40 ml Wasser aufgelöst, worauf man die Lösung auf 70°C erwärmt und mit 40 ml Aethanol versetzt. Die Lösung wird heiss filtriert, angeimpft und zum Kristallisieren in einem Kühlschrank stehengelassen. Man erhält 3,8 g D-α-Amino-2-imidazolpropionsäure vom Schmelzpunkt 240°C (Zersetzung), $[\alpha]_D^{20}$ = +27,6° (c = 1,08% in Wasser).

Analyse für $C_6H_9N_3O_2$:
Berechnet: C 46,45; H 5,85; N 27,1%.
Gefunden: C 46,7 ; H 5,8 ; N 27,2%.

Beispiel 19

Aethyl L-α-amino-2-imidazolpropionat-dihydrochlorid-trihydrat wird wie in Beispiel 18 beschrieben hydrolysiert, wobei man L-α-Amino-2-imidazolpropionsäure vom Schmelzpunkt 239°C (Zersetzung), erhält, $[\alpha]_D^{20}$ = -27,6° ( c = 1,00% in Wasser).

Analyse für $C_6H_9N_3O_2$:
Berechnet: C 46,45; H 5,85; N 27,1%.
Gefunden: C 46,2 ; H 5,7 ; N 27,0%.

Beispiel 20

Eine Mischung aus 1,48 g (9,55 mMol) D,L-α-Amino-2-imidazolpropionsäure, 3,8 g (20 mMol) p-Toluolsulfonsäuremonohydrat, 10 ml Benzylalkohol und 10 ml Chloroform wird in einer mit einem Soxhlet-Extraktor versehen Apparatur unter starkem Rückfluss zum Sieden erhitzt. Der Extraktor

wird mit einer Hülse, welche 12 g Molekularsieb (4A) enthält, und 22 ml Chloroform geladen. Man erhitzt die Mischung während 30 Stunden unter Rückfluss, wobei man das Molekularsieb nach 18 Stunden erneuert. Man engt die Mischung anschliessend unter vermindertem Druck ein und behandelt den gelben, gummiartigen Rückstand zweimal mit je 30 ml Diäthyläther, um überschüssigen Benzylalkohol zu entfernen. Das gummiartige Material wird dann in 30 ml Chloroform aufgenommen, und die Lösung wird dann mit 4 ml gesättigter Kaliumcarbonatlösung und 10 ml Wasser ausgeschüttelt. Beim Stehen entstehen drei Schichten, wovon die untere und die mittlere Schicht abgetrennt und mit 20 ml Wasser geschüttelt werden. Beim Stehenlassen entstehen lediglich zwei Schichten. Die untere Chloroformschicht wird abgetrennt und filtriert, worauf man das Filtrat über wasserfreiem Natriumsulfat trocknet und unter vermindertem Druck zu einem gelben Oel eindampft (2,0 g). Dieses Oel wird in 30 ml trockenem Tetrahydrofuran aufgenommen, die erhaltene Lösung wird in einer Eis/Salz-Mischung abgekühlt, worauf man solange Chlorwasserstoff durch diese Lösung leitet, bis kein weiterer Niederschlag entsteht und die Mischung stark sauer ist. Man dampft die Mischung unter vermindertem Druck zur Trockene ein, behandelt den Rückstand zweimal mit 20 ml trockenem Tetrahydrofuran und dampft erneut unter vermindertem Druck ein. Man suspendiert den erhaltenen gelben Festkörper in 20 ml Acetonitril, filtriert, wäscht zweimal mit je 10 ml Acetonitril und trocknet dann im Vakuum, wobei man 1,0 g eines Produktes erhält. Durch Umkristallisieren aus Wasser erhält man 0,5 g Benzyl D,L-α-amino-2-imidazolpropionat-dihydrochlorid in Form von weissen Kristallen mit einem Schmelzpunkt von 190-192°C.

Analyse für $C_{13}H_{15}N_3O_2.2HCl$:

Berechnet: C 49,1; H 5,4; N 13,2%.

Gefudnen: C 49,1; H 5,4; N 13,4%.

Beispiel 21

53 g D,L-α-Amino-α-methyl-1-benzyl-2-imidazolpropionsäure-dihydrochlorid werden in einem 2,5 l Vier-Hals-Rundkolben, der mit einem pneumatischen Rührer und einem Ammoniakkondenser versehen ist, vorgelegt und in einem Aceton/Trockeneis-Bad unter langsamem Rühren abgekühlt, worauf man 1 l Ammoniak in den Kolben kondensiert. Kleine, frisch geschnittene Natriumstücke (etwa 24 g) werden solange zugegeben bis eine permanente tiefblaue Farbe während etwa 20 Minuten bestehen bleibt. Anschliessend wird Ammoniumchlorid hinzugegeben, um die blaue Farbe zu zerstören. Man lässt dann das Ammoniak über Nacht abdampfen, versetzt den Rückstand mit etwa 60 ml Wasser und dampft die Mischung ein, um das während der Reaktion gebildete Toluol zu entfernen. Der Rückstand wird erneut in Wasser aufgenommen, und der pH der Lösung wird auf 8,5 eingestellt, und zwar mittels konzentrierter Salzsäure. Man dampft im Vakuum zur Trockene ein, löst den Rückstand in einem Minimum an heissem Wasser und lässt zum Kristallisieren stehen. Man erhält 13,6 g des Produktes mit einem Schmelzpunkt von 158-165°C. Nach Kristallisieren aus Wasser erhält man 4,8 g Produkt mit einem Schmelzpunkt von 160-170°C. Man nimmt dieses Produkt in Wasser auf und versetzt mit konzentrierter Salzsäure, bis die Lösung einen pH von 3 hat. Anschliessend dampft man im Vakuum zur Trockene ein, versetzt den Rückstand mit Toluol, dampft erneut ein, nimmt den erhaltenen Festkörper in Aethanol auf und lässt stehen. Das ausgefallene Material wird anschliessend abfiltriert. Man erhält 3,8 g D,L-α-Amino-α-methyl-2-imidazolpropionsäure-hydrochlorid vom Schmelzpunkt 264-267°C.

Analyse für $C_7H_{11}N_3O_2.HCl$:

Berechnet: C 40,9; H 5,9; N 20,4%.

Gefunden: C 40,8; H 6,0; N 20,2%.

Das als Ausgangsmaterial verwendete D,L-α-Amino-α-methyl-1-benzyl-2-imidazolpropionsäure-dihydrochlorid wird wie folgt hergestellt:

Man wäscht 14,5 g (483 mMol) Natriumhydrid (80-proz. in Oel) mit Petroläther (40-60°C), dekantiert den Petroläther ab, suspendiert das Natriumhydrid in 200 ml trockenem Dimethylformamid, kühlt die Suspension in einem Eisbad ab und versetzt tropfenweise mit 55 g (433 mMol) Aethyl 2-isocyanopropionat in 100 ml trockenem Dimethylformamid. Nach beendeter Zugabe lässt man die Mischung auf Raumtemperatur erwärmen und rührt sie bei dieser Temperatur während 1 Stunde. Anschliessend versetzt man portionenweise mit 53 g (218 mMol) 1-Benzyl-2-chlormethylimidazol-hydrochlorid und rührt die Mischung über Nacht bei Raumtemperatur. Man giesst die Mischung unter Rühren auf 1,5 l Wasser, extrahiert die wässrige Mischung fünfmal mit je 200 ml Essigester, wäscht die vereinigten Essigesterauszüge mit gesättigter Natriumchloridlösung und Wasser, trocknet über wasserfreiem Kaliumcarbonat und dampft ein, wobei man ein gelbes Oel erhält, das beim Stehen über Nacht kristallisiert. Die Kristalle werden in einem Abzug abfiltriert und mit 5-proz. Essigester/Hexan gewaschen. Man erhält 47 g Aethyl D,L-α-isocyano-α-methyl-1-benzyl-2-imidazolpropionat mit einem Schmelzpunkt von 66-68°C.

47 g Aethyl D,L-α-isocyano-α-methyl-1-benzyl-1-imidazolpropionat werden unter Rühren portionenweise zu 500 ml konzentrierter Salzsäure gegeben. Nach beendeter Zugabe wird die Mischung während 3 Stunden auf 100°C erwärmt und dann auf Raumtemperatur abgekühlt und zur Trockene eingedampft. Man erhält ein braunes Oel, das wiederholt zusammen mit Toluol eingedampft wird. Man erhält 53 g D,L-α-Amino-α-methyl-1-benzyl-2-imidazolpropionsäure-dihydrochlorid in Form eines sehr hygroskopischen braunen Schaumes, das in einem geschlossenen Behälter aufbewahrt werden muss. Dieser Schaum kann erwünschtenfalls aus Isopropylalkohol umkristallisiert werden, wobei man einen Festkörper vom Schmelzpunkt 225-228°C erhält.

Analyse für $C_{14}H_{17}N_3O_2.2HCl$:

Berechnet: C 50,6; H 5,8; N 12,65%.

Gefunden: C 50,8; H 5,6; N 12,6%.

Beispiel 22

Man versetzt eine gerührte und in einem Aceton/Trockeneisbad gekühlte Suspension von 11 g (40 mMol) D,L-α-Amino-1-benzyl-4,5-dimethyl-2-imidazolpropionsäure in 80-100 ml flüssigem Ammoniak über einen Zeitraum von 1 Stunde portionenweise mit Natriummetall, und zwar bis eine blaue Farbe während 20 Minuten erhalten bleibt (man benötigt 1,5 g Natrium). Anschliessend lässt man das Ammoniak abdampfen, nimmt den Rückstand in 30 ml Wasser auf und neutralisiert durch Zugabe von Eisessig auf pH 7. Das ausgefallene kristalline Material wird abfiltriert, nacheinander mit Wasser, Aethanol und Diäthyläther gewaschen und getrocknet. Man erhält 2,4 g Material mit einem Schmelzpunkt von 213-214°C (Zersetzung). Durch Umkristallisieren aus Wasser erhält man D,L-α-Amino-4,5-dimethyl-2-imidazolpropionsäure vom Schmelzpunkt 217-218°C (Zersetzung). Nach Trocknen bei 90°C/0,1 mm während 4 Stunden schmilzt das Produkt bei 169-170°C.

Analyse für $C_8H_{13}N_3O_2 . o,2H_2O$:
Berechnet: C 51,4; H 7,2; N 22,5; $H_2O$ 1,9%.
Gefunden: C 51,4; H 7,2; N 22,5; $H_2O$ 2,25%.

Die als Ausgangsmaterial verwendete D,L-α-Amino-1-benzyl-4,5-dimethyl-2-imidazolpropionsäure wird wie folgt hergestellt:

Eine Mischung aus 8 g (43 mMol) 1-Benzyl-4,5-dimethylimidazol und 60 ml 40-proz. wässrige Formaldehydlösung wird in einem geschlossenen Autoklaven während 20 Stunden auf 150°C erhitzt. Man dampft die Mischung ein und chromatographiert den halbfesten Rückstand an 300 g Kieselgel unter Eluieren mit einer 20-proz. Lösung von Methanol in Chloroform. Nach Eindampfen der Eluate erhält man 6 g 1-Benzyl-4,5-dimethyl-2-hydroxymethylimidazol vom Schmelzpunkt 156-158°C. Nach Umkristallisieren aus Methylcyclohexan schmilzt das Produkt bei 160-162°C. Eine Portion des Produktes wird mit einer Lösung von Chlorwasserstoff in Aethanol behandelt.

Durch Zugabe von Diäthyläther fällt 1-Benzyl-4,5-dimethyl-2-hydroxymethylimidazol-hydrochlorid vom Schmelzpunkt 162-163°C aus.

Analyse für $C_{13}H_{16}N_2O.HCl$:

Berechnet: C 61,8; H 6,8; N 11,1%.

Gefunden: C 61,6; H 6,7; N 11,15%.

Eine Suspension von 15 g (69 mMol) 1-Benzyl-4,5-dimethyl-2-hydroxymethylimidazol in 50 ml Methylenchlorid wird über einen Zeitraum von 30 Minuten portionenweise zu einer gerührten Lösung von 25 ml Thionylchlorid in 25 ml Methylenchlorid gegeben. Die erhaltene Lösung wird während 1,5 Stunden unter Rückfluss zum Sieden erhitzt und dann über Nacht bei Raumtemperatur stehengelassen. Durch Eindampfen der Lösung erhält man 23,5 g rohes 1-Benzyl-2-chlormethyl-4,5-dimethylimidazol-hydrochlorid in Form eines Oeles, das man ohne weitere Reinigung in die nächste Stufe einsetzt.

Man gibt 40 g (184 mMol) Diäthylacetamidomalonat zu einer Lösung von 6,3 g (0,27 g Atom) Natrium in 250 ml Aethanol. Man rührt die erhaltene Lösung während 20 Minuten, kühlt sie dann in einem Eisbad ab und behandelt sie mit einer Lösung von 23,5 g rohem 1-Benzyl-2-chlormethyl-4,5-dimethylimidazol-hydrochlorid in 40 ml Aethanol. Die Mischung wird über Nacht bei Raumtemperatur gerührt und dann eingedampft. Den Rückstand nimmt mit in 300 ml 2N-Salzsäure auf, filtriert die erhaltene Lösung und schüttelt sie so oft mit 100 ml Portionen Essigester, bis kein Diäthylacetamidomalonat mehr extrahiert wird. Die wässrige Lösung wird dann mit einem Ueberschuss an festem Natriumcarbonat behandelt. Das ausgeschiedene Oel wird dann mit Essigester extrahiert, die Essigesterlösung wird über Magnesiumsulfat getrocknet und eingedampft. Man erhält 15,4 g rohes Diäthyl 1-benzyl-4,5-dimethyl-2-imidazolylmethylacetamidomalonat in Form eines Oels, das kristallisiert. Dieses Material wird ohne weitere Reinigung in die nächste Stufe eingesetzt.

Eine Lösung von 34 g rohem Diäthyl 1-benzyl-4,5-dimethyl-2-imidazolylmethylacetamidomalonat in 170 ml konzentrierter Salzsäure wird während 15 Stunden bei 130°C unter Rückfluss erhitzt. Man dampft die Mischung ein, behandelt den Rückstand mit Wasser und dampft erneut ein. Den so erhaltenen Rückstand nimmt man in 100 ml Wasser auf, neutralisiert die Lösung durch Zugabe von 2N-Natriumhydroxidlösung auf pH 7,2, lässt im Kühlschrank über Nacht stehen, filtriert den ausgefallenen Festkörper ab, wäscht diesen mit Wasser, Aceton und Diäthyläther und trocknet ihn. Man erhält 11,5 g Produkt vom Schmelzpunkt 224-225°C (Zersetzung). Dieser wird gereinigt, indem man ihn in verdünntem Ammoniak löst und die Lösung mit verdünnter Essigsäure auf pH 7,5 neutralisiert. Man erhält D,L-α-Amino-1-benzyl-4,5-dimethyl-2-imidazolpropionsäure vom Schmelzpunkt 224-225°C (Zersetzung).

Analyse für $C_{15}H_{19}N_3O_2$:
Berechnet: C 65,9; H 7,0; N 15,4%.
Gefunden: C 65,7; H 6,9; N 15,4%.

Beispiel 23

Eine Suspension von 5 g Methyl D,L-α-amino-2-imidazolpropionat in 50 ml trockenem Methanol wird unter Feuchtigkeitsausschluss und Kühlen in einer Eis/Salz-Mischung gerührt. Dimethylamingas wird dann so rasch in die feuchte Suspension eingeleitet, dass nach etwa 15 Minuten eine klare Lösung entsteht; die Lösung ist nach 30 Minuten praktisch gesättigt. Die Gaseinleitung wird dann beendet, worauf man die Lösung noch während weiteren 2 Stunden rührt und langsam auf Raumtemperatur erwärmen lässt. Man lässt dann bei Raumtemperatur über Nacht stehen. Falls auf dieser Stufe noch Methylester vorhanden ist (wird bestimmt, indem man ein Aliquot dieser Lösung zur Trockene eindampft und den Rückstand mittels magnetischer Kernresonanzspektroskopie untersucht), so wird das Durchleiten von Dimethylamin unter Kühlen mit Eis/Salz und das Stehenlassen über Nacht bei Raumtemperatur wiederholt. Die Lösung wird dann unter

vermindertem Druck bei 40°C zur Trockene eingedampft, worauf man den Rückstand in 40 ml Wasser aufnimmt. Ein kleiner Anteil an unlöslichem Material wird mittels Filtration entfernt, und das Filtrat wird auf eine Säule gegeben, welche 50 ml eines Ionenaustauscherharzes (Dowex 1-X8, $OH^-$-Form) gegeben. Die Säule wird solange mit Wasser extrahiert, bis der pH des Eluates weniger als 8 beträgt. Das Eluat (250 ml) wird bei 40°C unter vermindertem Druck eingedampft, wobei man ein hellgelbes Oel erhält, das beim Stehen kristallisiert. Dieses Material wird in 20 ml warmem Aethanol aufgenommen, worauf man mit einer Lösung von Chlorwasserstoff in Aethanol (10 ml; 5,7N) versetzt und die stark saure Lösung in einem Kühlschrank über Nacht stehen lässt. Das auskristallisierte Produkt wird abfiltriert, mit Aethanol und Diäthyläther gewaschen und bei 50°C im Vakuum über Phosphorpentoxid getrocknet, wobei man 5 g Produkt mit einem Schmelzpunkt von 223-224°C (Zersetzung) erhält. Man reinigt dieses Material, indem man es in 5 ml warmem Wasser aufnimmt, 20 ml Aethanol dazugibt, die Lösung filtriert, das Filtrat mit 80 ml Aethanol verdünnt und die Lösung bei Raumtemperatur während 4 Stunden zum Kristallisieren stehen lässt. Anschliessend lässt man noch in einem Kühlschrank über Nacht stehen. Das weisse kristalline Produkt wird abfiltriert, mit Aethanol und Diäthyläther gewaschen und dann bei 50°C im Vakuum über Phosphorpentoxid getrocknet, wobei man 4,0 g D,L-α-Amino-N,N-dimethyl-2-imidazolpropionamid-dihydrochlorid vom Schmelzpunkt 231-232°C (Zersetzung) erhält.

Analyse für $C_8H_{14}N_4O.2HCl$:
Berechnet: C 37,7; H 6,3; Cl 27,8; N 22,0%.
Gefunden: C 37,5; H 6,25; Cl 27,9; N 22,0%.

Beispiel 24

Aus 5 g Methyl D,L-α-amino-2-imidazolpropionat und Methylamin erhält man in Analogie zu Beispiel 23 3,65 g reines D,L-α-Amino-N-methyl-2-imidazolpropionamid-dihydrochlorid in Form von weissen hydratisierten Kristallen mit

einem Schmelzpunkt von 191-193°C.
Analyse für $C_7H_{12}N_4O.2HCl.0,6H_2O$:

Berechnet: C 33,4; H 6,1; Cl 28,1; N 22,2%.

Gefunden: C 33,3; H 6,1; Cl 27,9; N 22,4%.

Beispiel 25

Aus 5 g Methyl D,L-α-amino-2-imidazolpropionat und trockenem Ammoniak erhält man in Analogie zu Beispiel 23 4 g reines D,L-α-Amino-2-imidazolpropionamid-dihydrochlorid in Form von weissen Kristallen vom Schmelzpunkt 215-217°C (Zersetzung).
Analyse für $C_6H_{10}N_4O.2HCl$:

Berechnet: C 31,7; H 5,3; Cl 31,2; N 24,7%.

Gefunden: C 31,7; H 5,3; Cl 31,3; N 24,8%.

Beispiel 26

(A) 10 g Benzyl D-α-t-butoxycarbonylamino-1-t-butoxycarbonyl-2-imidazolpropionat werden in 400 ml einer 4N-Lösung von Chlorwasserstoff in Dioxan gelöst, worauf die erhaltene gelbe Lösung während 2 Stunden bei Raumtemperatur gerührt wird. Man konzentriert die Lösung bei 30°C unter vermindertem Druck auf etwa 3/4 ihres ursprünglichen Volumens ein, worauf ein gummiartiges Material ausfällt. Man versetzt mit 500 ml wasserfreiem Diäthyläther und lässt die Mischung während 2 Stunden in einem Kühlschrank stehen. Die überstehende gelbe, gummiartige Flüssigkeit wird abdekantiert und noch einmal mit 250 ml Diäthyläther behandelt. Der so erhaltene Festkörper wird abfiltriert, zweimal mit je 30 ml Diäthyläther gewaschen und getrocknet. Durch Aufarbeiten der Mutterlaugen erhält man zwei weitere Portionen Produkt. Die Gesamtausbeute an Benzyl D-α-amino-2-imidazolpropionat-dihydrochlorid, das an der Luft zerfliesst, beträgt 4,75 g.

Das obige Dihydrochlorid wurde wie folgt in das Monohydrochlorid übergeführt:

2,4 g (7,55 mMol) Dihydrochlorid werden mit 15 ml gesättigter Kaliumcarbonatlösung, 10 ml Wasser und 25 ml Chloroform geschüttelt, worauf man die Schichten trennt und die wässrige Phase dreimal mit je 10 ml Chloroform extrahiert. Die vereinigten organischen Auszüge werden getrocknet und unter vermindertem Druck zur Trockene eingedampft, wobei man 2 g rohes Benzyl D-α-amino-2-imidazolpropionat in Form eines braunen Oeles erhält. 1,84 g dieses Oeles werden in 50 ml Isopropanol gelöst und zu einer Lösung von 2,2 g (6,92 mMol) Benzyl D-α-amino-2-imidazolpropionat-dihydrochlorid in 50 ml Isopropanol gegeben. Die Lösung wird anschliessend filtriert, worauf man das Filtrat animpft, bei Raumtemperatur und unter vermindertem Druck auf die Hälfte des Volumens eindampft und über Nacht bei -20°C stehen lässt. Das ausgefallene weisse, kristalline Monohydrochlorid wird abfiltriert, nacheinander mit 10 ml kaltem Isopropanol und mit 20 ml Diäthyläther gewaschen und getrocknet. Man erhält 2,80 g (72%) Benzyl D-α-amino-2-imidazolpropionat-monohydrochlorid vom Schmelzpunkt 114-117°C, $[\alpha]_D^{20}$ = -18,5° (c = 1,0004% in 1N-Salzsäure).

Analyse für $C_{13}H_{15}N_3O_2$.HCl:

Berechnet: C 55,4; H 5,7; Cl 12,6; N 14,9%.

Gefunden: C 55,3; H 5,8; Cl 12,7, N 14,7%.

Das als Ausgangsmaterial verwendete Benzyl D-α-t-butoxycarbonylamino-1-t-butoxycarbonyl-2-imidazolpropionat wird wie folgt hergestellt:

(B) Man rührt eine Mischung aus 4,65 g (30 mMol) D-α-Amino-2-imidazolpropionsäure, 60 ml 1N-Natriumhydroxid und 60 ml Dioxan, kühlt die erhaltene Lösung in einem Eisbad ab und versetzt über einen Zeitraum von 10 Minuten portionenweise mit 16,35 g (75 mMol) Di-t-butyldicarbonat Nach 30 Minuten ist der pH von oberhalb 11 auf etwa 8 und nach weiteren 2 Stunden auf etwa 7 gefallen. Das Kühlbad wird dann entfernt, worauf man 5,04 g (60 mMol) festes Natriumhydrogencarbonat hinzugibt und die Mischung über Nacht bei Raumtemperatur rührt. Die erhaltene Suspension wird filtriert,

und der Festkörper wird in 90 ml Wasser aufgenommen. Durch Filtrieren entfernt man eine geringfügige Menge an unlöslichem Material. Die vereinigten Filtrate werden zweimal mit je 150 ml Diäthyläther gewaschen und unter Rühren in einem Eisbad abgekühlt, worauf man 33 g feste Zitronensäure vorsichtig hinzugibt, um den pH auf 3 einzustellen. Die kalte Mischung wird viermal mit je 90 ml Diäthyläther extrahiert, die vereinigten Extrakte werden filtriert, über wasserfreiem Natriumsulfat getrocknet und bei einer Temperatur unterhalb 30°C zur Trockene eingedampft. Man erhält 10,5 g (98%) D-α-t-Butoxycarbonylamino-1-t-butoxycarbonyl-2-imidazolpropionsäure in Form eines farblosen Gummis, das beim Stehenlassen kristallisiert.

Eine ätherische Lösung von Phenyldiazomethan (300 ml, enthaltend 4,18 g Phenyldiazomethan, 35,4 mMol) wird über einen Zeitraum von 15 Minuten tropfenweise zu einer gerührten Suspension von 10,5 g (29,6 mMol) D-α-t-Butoxycarbonylamino-1-t-butoxycarbonyl-2-imidazolpropionsäure in 500 ml Diäthyläther gegeben. Man rührt die Mischung während 2 Stunden, wobei der Festkörper innerhalb der ersten Stunde in Lösung geht, und lässt dann bei Raumtemperatur während 2 Stunden stehen. Die orangfarbene ätherische Lösung wird mit 500 ml 2M-Zitronensäure solange geschüttelt, bis die Farbe auf hellgelb wechselt. Anschliessend wäscht man zweimal mit je 500 ml 20-proz. Kaliumcarbonatlösung und trocknet über Natriumsulfat. Nach Eindampfen im Vakuum erhält man 10,0 g (76%) Benzyl D-α-t-butoxycarbonylamino-1-t-butoxycarbonyl-2-imidazolpropionat in Form eines gelben Oeles.

## Beispiel 27

Aus Benzyl L-α-t-butoxycarbonylamino-1-t-butoxycarbonyl-2-imidazolpropionat erhält man in Analogie zu Beispiel 26(A) Benzyl L-α-amino-2-imidazolpropionat-monohydrochlorid vom Schmelzpunkt 114-117°C, $[\alpha]_D^{20}$ = +18,6° (c = 0,996% in 1N-Salzsäure).

Analyse für $C_{13}H_{15}N_3O_2.HCl$:
Berechnet: C 55,4; H 5,7; Cl 12,6; N 14,9%.
Gefunden: C 55,3; H 5,7; Cl 12,7; N 14,8%.

Das als Ausgangsmaterial verwendete Benzyl L-α-t-butoxycarbonylamino-1-t-butoxycarbonyl-2-imidazolpropionat wird in Analogie zu Beispiel 26(B) aus L-α-Amino-2-imidazol-propionsäure hergestellt.

Patentansprüche

1. Pharmazeutische Präparate enthaltend als Wirkstoff eine D,L-, D- oder L-Verbindung der allgemeinen Formel

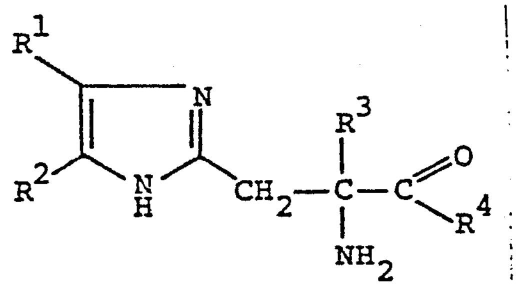

I

worin $R^1$, $R^2$ und $R^3$ je Wasserstoff oder niederes Alkyl und $R^4$ Hydroxy, niederes Alkoxy, Aryl-niederes Alkoxy, Amino, Mono(niederes Alkyl)amino oder Di(niederes Alkyl)amino bedeuten,
oder ein pharmazeutisch annehmbares Salz davon.

2. Pharmazeutische Präparate gemäss Anspruch 1, dadurch gekennzeichnet, dass sie eine D,L-, D- oder L-Verbindung der Formel I, worin $R^1$, $R^2$ und $R^3$ je Wasserstoff und $R^4$ niederes Alkoxy bedeuten, oder ein pharmazeutisch annehmbares Salz davon enthalten.

3. Pharmazeutische Präparate gemäss Anspruch 2, dadurch gekennzeichnet, dass sie D-α-Amino-2-imidazolpropionat oder ein pharmazeutisch annehmbares Salz davon enthalten.

4. Pharmazeutische Präparate gemäss einem der Ansprüche 1-3 für die Behandlung von degenerativen Gelenkkrankheiten und der Wilson-Krankheit.

5. D,L-, D- und L-Verbindungen der allgemeinen Formel

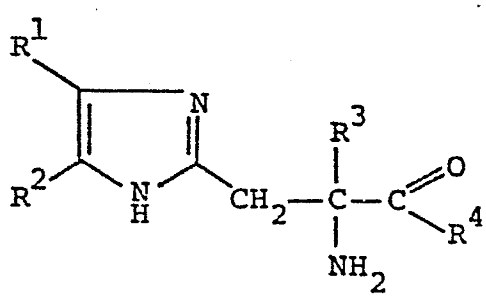

I

worin $R^1$, $R^2$ und $R^3$ je Wasserstoff oder niederes Alkyl und $R^4$ Hydroxy, niederes Alkoxy, Aryl-niederes Alkoxy, Amino, Mono(niederes Alkyl)amino oder Di(niederes Alkyl)amino bedeuten, mit Ausnahme der D,L-Verbindungen der Formel I, worin $R^1$, $R^2$ und $R^3$ je Wasserstoff und $R^4$ Hydroxy oder Methoxy bedeuten, und pharmazeutisch annehmbare Salze davon.

6. Verbindungen gemäss Anspruch 5, worin $R^4$ eine von Hydroxy oder Methoxy verschiedene Bedeutung hat, wenn $R^1$, $R^2$ und $R^3$ je Wasserstoff bedeuten, und pharmazeutisch annehmbare Salze davon.

7. Verbindungen gemäss Anspruch 5 oder 6, worin $R^1$, $R^2$ und $R^3$ je Wasserstoff und $R^4$ niederes Alkoxy mit mindestens 2 Kohlenstoffatomen bedeuten, und pharmazeutisch annehmbare Salze davon.

8. Aethyl D-α-amino-2-imidazolpropionat und pharmazeutisch annehmbare Salze davon.

9. Eine Verbindung aus der Gruppe, bestehend aus
D-α-Amino-2-imidazolpropionsäure,
L-α-Amino-2-imidazolpropionsäure,
Methyl L-α-amino-2-imidazolpropionat,
Methyl D-α-amino-2-imidazolpropionat,
Aethyl D,L-α-amino-2-imidazolpropionat,
Aethyl L-α-amino-2-imidazolpropionat,
Benzyl D,L-α-amino-2-imidazolpropionat,
D,L-α-Amino-α-methyl-2-imidazolpropionsäure,
D,L-α-Amino-4,5-dimethyl-2-imidazolpropionsäure,
D,L-α-Amino-2-imidazolpropionamid,
D,L-α-Amino-N-methyl-2-imidazolpropionamid und
D,L-α-Amino-N,N-dimethyl-2-imidazolpropionamid,
und pharmazeutisch annehmbare Salze davon.

10. Eine Verbindung aus der Gruppe, bestehend aus Benzyl D-α-amino-2-imidazolpropionat und Benzyl L-α-amino-2-imidazolpropionat, und pharmazeutisch annehmbare Salze davon.

11. D,L-, D- und L-Verbindungen der allgemeinen Formel

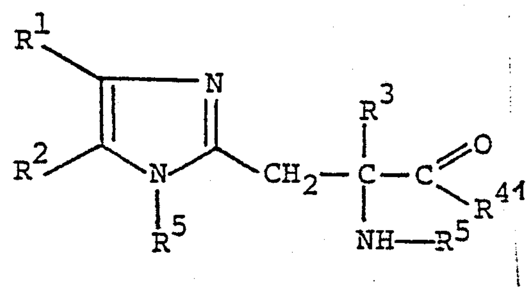

III

worin $R^1$, $R^2$ und $R^3$ die in Anspruch 1 angegebene Bedeutung besitzen und $R^{41}$ Hydroxy, niederes Alkoxy oder Aryl—niederes Alkoxy und $R^5$ t-Butoxycarbonyl bedeuten.

12. Verfahren zur Herstellung einer Verbindung gemäss einem der Ansprüche 5-10, dadurch gekennzeichnet, dass man

a) zur Herstellung einer D,L-Verbindung der Formel I, worin mindestens eines von $R^1$, $R^2$ und $R^3$ niederes Alkyl und $R^4$ Hydroxy bedeuten, eine D,L-Verbindung der allgemeinen Formel

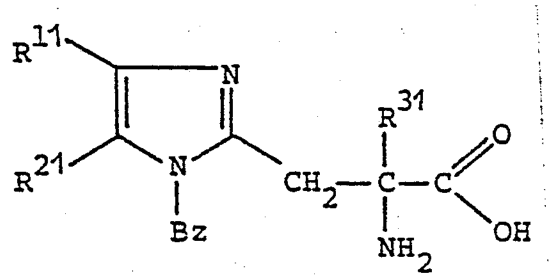

II

worin $R^{11}$, $R^{21}$ und $R^{31}$ je Wasserstoff oder niederes Alkyl und Bz Benzyl bedeuten, wobei mindestens eines von $R^{11}$, $R^{21}$ und $R^{31}$ niederes Alkyl bedeutet, debenzyliert, oder

b) zur Herstellung einer D,L-, D- oder L-Verbindung der Formel I, worin $R^4$ niederes Alkoxy mit mindestens 2 Kohlenstoffatomen bedeutet, oder einer D- oder L-Verbindung der Formel I, worin $R^4$ Methoxy bedeutet, oder einer D,L-Verbindung der Formel I, worin $R^4$ Aryl-niederes Alkoxy bedeutet, eine entsprechende D,L-, D- oder L-Verbindung der Formel I, worin $R^4$ Hydroxy bedeutet, verestert, oder

c) zur Herstellung einer D,L-, D- oder L-Verbindung der Formel I, worin $R^4$ Amino, Mono(niederes Alkyl)amino oder Di(niederes Alkyl)amino bedeutet, eine D,L-, D- oder L-Verbindung der Formel I, worin $R^4$ Hydroxy oder niederes Alkoxy bedeutet, entsprechend amidiert, oder

d) zur Herstellung einer D,L-, D- oder L-Verbindung der Formel I, worin $R^4$ niederes Alkoxy mit mindestens 2 Kohlenstoffatomen oder Aryl-niederes Alkoxy bedeutet, oder einer D- oder L-Verbindung der Formel I, worin $R^4$ Methoxy bedeutet, eine D,L-, D- oder L-Verbindung der allgemeinen Formel

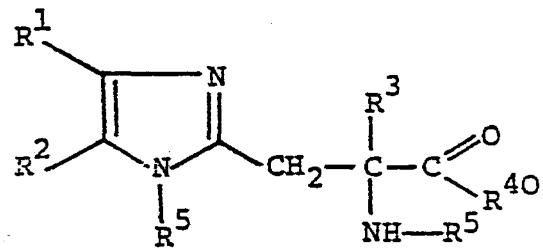

IIIa

worin $R^1$, $R^2$ und $R^3$ obige Bedeutung besitzen, $R^{40}$ niederes Alkoxy oder Aryl—niederes Alkoxy und $R^5$ t-Butoxycarbonyl bedeuten,

hydrolysiert, oder

e) zur Herstellung einer D- oder L-Verbindung der Formel I, worin $R^4$ Hydroxy bedeutet, oder einer D,L-Verbindung der Formel I, worin mindestens eines von $R^1$, $R^2$ und $R^3$ niederes Alkyl und $R^4$ Hydroxy bedeuten, eine entsprechende D-, L- oder D,L-Verbindung der Formel I, worin $R^4$ niederes Alkoxy bedeutet, hydrolysiert, oder

f) zur Herstellung einer D- oder L-Verbindung der Formel I, eine D,L-Verbindung der Formel I einer Racematspaltung

unterwirft, und die D- oder L-Verbindung isoliert, und erwünschtenfalls eine erhaltene Verbindung der Formel I in ein pharmazeutisch annehmbares Salz überführt.

13. D,L-, D- und L-Verbindungen der Formel I gemäss Anspruch 1 oder 2, und pharmazeutisch annehmbare Salze davon als pharmazeutische Wirkstoffe.

14. D,L-, D- oder L-Verbindungen der Formel I gemäss Anspruch 1 oder 2 und pharmazeutisch annehmbare Salze davon als pharmazeutische Wirkstoffe für die Behandlung von degenerativen Gelenkkrankeiten und der Wilson-Krankheit.

15. Aethyl D-α-amino-2-imidazolpropionat und pharmazeutisch annehmbare Salze davon als pharmazeutische Wirkstoffe.

16. Aethyl D-α-amino-2-imidazolpropionat und pharmazeutisch annehmbare Salze davon als pharmazeutische Wirkstoffe zur Behandlung von degenerativen Gelenkkrankheiten und der Wilson-Krankheit.

***

Patentansprüche für Oesterreich

1. Verfahren zur Herstellung von D,L-, D- und L-Verbindungen der allgemeinen Formel

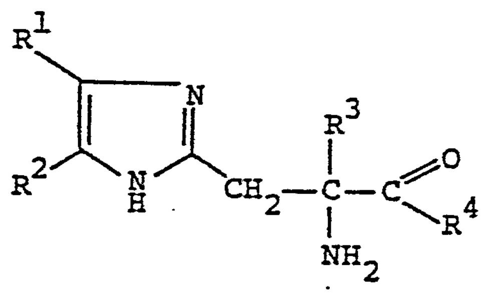

I

worin $R^1$, $R^2$ und $R^3$ je Wasserstoff oder niederes Alkyl und $R^4$ Hydroxy, niederes Alkoxy, Aryl-niederes Alkoxy, Amino, Mono(niederes Alkyl)amino oder Di(niederes Alkyl)amino bedeuten, mit Ausnahme der D,L-Verbindungen der Formel I, worin $R^1$, $R^2$ und $R^3$ je Wasserstoff und $R^4$ Hydroxy oder Methoxy bedeuten, und pharmazeutisch annehmbaren Salzen davon, dadurch gekennzeichnet, dass man

a) zur Herstellung einer D,L-Verbindung der Formel I, worin mindestens eines von $R^1$, $R^2$ und $R^3$ niederes Alkyl und $R^4$ Hydroxy bedeuten, eine D,L-Verbindung der allgemeinen Formel

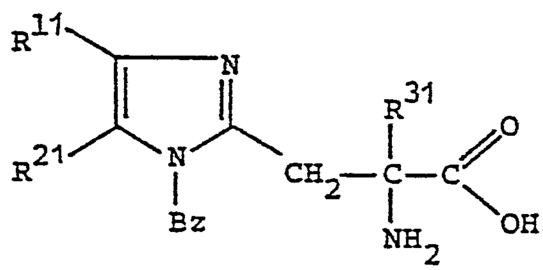

II

worin $R^{11}$, $R^{21}$ und $R^{31}$ je Wasserstoff oder niederes Alkyl und Bz Benzyl bedeuten, wobei mindestens eines von $R^{11}$, $R^{21}$ und $R^{31}$ niederes Alkyl bedeutet, debenzyliert, oder

b) zur Herstellung einer D,L-, D- oder L-Verbindung der Formel I, worin $R^4$ niederes Alkoxy mit mindestens 2 Kohlenstoffatomen bedeutet, oder einer D- oder L-Verbindung

der Formel I, worin $R^4$ Methoxy bedeutet, oder einer D,L-Verbindung der Formel I, worin $R^4$ Aryl-niederes Alkoxy bedeutet, eine entsprechende D,L-, D- oder L-Verbindung der Formel I, worin $R^4$ Hydroxy bedeutet, verestert, oder

c) zur Herstellung einer D,L-, D- oder L-Verbindung der Formel I, worin $R^4$ Amino, Mono(niederes Alkyl)amino oder Di(niederes Alkyl)amino bedeutet, eine D,L-, D- oder L-Verbindung der Formel I, worin $R^4$ Hydroxy oder niederes Alkoxy bedeutet, entsprechend amidiert, oder

d) zur Herstellung einer D,L-, D- oder L-Verbindung der Formel I, worin $R^4$ niederes Alkoxy mit mindestens 2 Kohlenstoffatomen oder Aryl-niederes Alkoxy bedeutet, oder einer D- oder L-Verbindung der Formel I, worin $R^4$ Methoxy bedeutet, eine D,L-, D- oder L-Verbindung der allgemeinen Formel

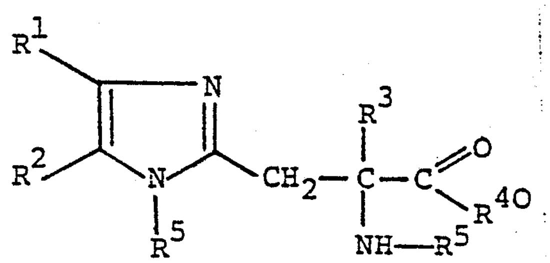

IIIa

worin $R^1$, $R^2$ und $R^3$ obige Bedeutung besitzen, $R^{40}$ niederes Alkoxy oder Aryl—niederes Alkoxy und $R^5$ t-Butoxycarbonyl bedeuten,

hydrolysiert, oder

e) zur Herstellung einer D- oder L-Verbindung der Formel I, worin $R^4$ Hydroxy bedeutet, oder einer D,L-Verbindung der Formel I, worin mindestens eines von $R^1$, $R^2$ und $R^3$ niederes Alkyl und $R^4$ Hydroxy bedeuten, eine entsprechende D-, L- oder D,L-Verbindung der Formel I, worin $R^4$ niederes Alkoxy bedeutet, hydrolysiert, oder

f) zur Herstellung einer D- oder L-Verbindung der Formel I, eine D,L-Verbindung der Formel I einer Racematspaltung unterwirft, und die D- oder L-Verbindung isoliert, und

erwünschtenfalls eine erhaltene Verbindung der Formel I in ein pharmazeutisch annehmbares Salz überführt.

2. Verfahren gemäss Anspruch 1, dadurch gekennzeichnet, dass man die Verfahren a), b), c) und f) verwendet.

3. Verfahren gemäss Anspruch 1, dadurch gekennzeichnet, dass man das Verfahren d) verwendet.

4. Verfahren gemäss Anspruch 1, dadurch gekennzeichnet, dass $R^4$ eine von Hydroxy und Methoxy verschiedene Bedeutung hat, wenn $R^1$, $R^2$ und $R^3$ je Wasserstoff bedeuten.

5. Verfahren gemäss Anspruch 1 oder 4, dadurch gekennzeichnet, dass $R^1$, $R^2$ und $R^3$ je Wasserstoff und $R^4$ niederes Alkoxy mit mindestens 2 Kohlenstoffatomen bedeuten.

6. Verfahren gemäss Anspruch 1, dadurch gekennzeichnet, dass man Aethyl D-α-amino-2-imidazolpropionat oder ein pharmazeutisch annehmbares Salze davon herstellt.

Europäisches Patentamt

# EUROPÄISCHER RECHERCHENBERICHT

0118787
Nummer der Anmeldung

EP 84 10 1489

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | JOURNAL OF THE AMERICAN CHEMICAL SOCIETY, Band 71, Nr. 2, 28. Februar 1949, Seiten 383-386, Washington, D.C., USA<br>R.G. JONES: "Studies on imidazole compounds. I. A synthesis of imidazoles with functional groups in the 2-position" * Seiten 383,384,386 *<br>--- | 9,12 | C 07 D 233/64<br>A 61 K 31/415 |
| X | JOURNAL OF MEDICINAL CHEMISTRY, Band 15, Nr. 12, December 1972, Seiten 1259-1261, Washington, D.C., USA<br>G.E. TROUT: "Synthesis of some histidine analogs and their effect on the growth of a histidine-requiring mutant of Leuconostoc mesenteroides" * Seiten 1259-1261 *<br>----- | 9 | |
| | | | RECHERCHIERTE SACHGEBIETE (Int. Cl. 3) |
| | | | C 07 D 233/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 15-05-1984 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82